# FASCICULE DE BREVET EUROPEEN

(11) **EP 0 670 164 B1**
(45) Date de publication et mention de la délivrance du brevet: **23.01.2002**
(21) Numéro de dépôt: 94402845.5
(22) Date de dépôt: 09.12.1994
(51) Int. Cl.: A61K 31/70

(54) **Solutions de type homéopathique contenant un acide nucléique**
Eine Nukleinsäure enthaltende homöopatische Lösung
Homeopathic solutions containing a nucleic acid

(30) Priorité: 09.12.1993 FR 9314796
(43) Date de publication de la demande: 06.09.1995
(73) Titulaire: LABO'LIFE, 79160 La Chapelle Thireuil (FR)
(72) Inventeur: Marichal, Bernard, B-1040 Bruxelles (BE); Jenaer, Maurice, B-1150 Bruxelles (BE)
(74) Mandataire: Stalla-Bourdillon, Bernard

(56) Documents cités:
- EP-A- 0 278 876
- FR-A- 2 592 790
- FR-A- 2 680 687
- FR-A- 2 699 820

## Description

L'invention a pour objet un procédé de préparation de solutions de type homéopathique par utilisation d'acides nucléiques spécifiques, les médicaments obtenus à partir desdites solutions ainsi que l'utilisation desdites solutions dans la préparation de compositions destinées à prévenir ou à traiter diverses maladies.

Le traitement de maladies par l'administration à doses pondérales de composés choisis pour leurs effets opposés à la cause ou aux symptômes de la maladie traitée, s'est avérée présenter différents inconvénients. L'efficacité s'est révélée relativement limitée dans le traitement d'un certain nombre de maladies qui, dès lors, sont considérées comme incurables, présentant par exemple des manifestations récurrentes pouvant aller de la simple gêne pour le malade à un réel danger pour sa vie. Ces maladies peuvent être de différents types tels qu'infectieux et notamment viral comme le syndrome d'immunodéficience acquise (SIDA) ou l'herpès, de type héréditaire comme notamment la mucoviscidose ou la myopathie de Duchenne ou bien encore de type cancéreux. En outre, dans de très nombreux autres cas, si le traitement se révèle efficace sur la maladie proprement dite, son administration est si astreignante ou ledit traitement présente de tels effets secondaires que le bien-être du malade s'en trouve considérablement affecté.

De plus, il est également souhaitable de pouvoir prévenir ou retarder l'apparition d'un certain nombre de maladies. De même que dans le domaine curatif, les techniques usuelles dans le domaine préventif se sont avérées dans un certain nombre de cas peu satisfaisantes.

Divers procédés ont été envisagés en vue d'éviter notamment les effets secondaires présentés par ces traitements.

Parmi ceux-ci, on peut citer la haute dilution de composés actifs, telles que pratiquées dans les préparations homéopathiques. Ainsi, la Directive 92/73 du Conseil des Communautés Européennes (Article 7, al. 1) garantit l'inocuité des médicaments ne contenant "ni plus d'une partie par 10000 de la teinture mère, ni plus d'un centième de la plus petite dose utilisée éventuellement en allopathie pour les principes actifs dont la présence dans un médicament allopathique entraîne l'obligation de présenter une prescription médicale".

Il a également été décrit dans la demande FR-A-2680 687 des compositions homéopathiques ainsi que leur utilisation pour la préparation de médicaments destinés à traiter, entre autres, certaines affections virales. Ces compositions contiennent une dilution homéopathique de la particule virale entière ou un fragment de cette particule tel que l'ADN ou l'ARN viral, une protéine virale, ou un fragment de ceux-ci, afin de provoquer l'élimination par la cellule de ces particules virales.

On a maintenant constaté de façon surprenante et inattendue qu'en utilisant une solution hautement diluée et dynamisée d'un acide nucléique spécifique, on pouvait obtenir un médicament efficace ne présentant pas les inconvénients décrits précédemment.

La présente invention a pour objet un procédé de préparation d'une solution de type homéopathique destinée à traiter ou à prévenir une maladie infectieuse ou impliquant le dysfonctionnement d'un gène, chez un individu d'une espèce donnée, comprenant les étapes consistant à :
i) mettre en solution dans un solvant pharmaceutiquement acceptable, dans une proportion correspondant à environ une nanomole pour 10 ml de solvant, un acide nucléique simple brin de longueur au moins égale à 10 nucléotides, et de séquence sensiblement analogue à celle d'un polynucléotide choisi soit parmi une partie du matériel génomique de l'agent infectieux, le produit de transcription de ladite partie ou le cas échéant le produit de transcription inverse de ladite partie, lorsque ladite maladie à traiter ou à prévenir est du type infectieux, soit parmi une partie du matériel génétique de ladite espèce, comprenant une partie de la séquence nucléotidique liée audit dysfonctionnement ou une partie du produit de transcription de ladite séquence liée au dysfonctionnement, lorsque ladite maladie à traiter ou à prévenir implique le dysfonctionnement d'un gène, ledit acide nucléique étant de l'ADN lorsque ledit polynucléotide est de l'ARN ou étant de l'ARN lorsque ledit polynucléotide est de l'ADN, à condition que lorsque l'agent infectieux est un virus à ARN, ledit acide nucléique ne soit pas l'ARN viral ou l'un de ses fragments, et que lorsque l'agent infectieux est un virus à ADN, ledit acide nucléique ne soit pas l'ADN viral ou l'un de ses fragments,
ii) dynamiser ladite solution par succussion,
iii) diluer d'un facteur pouvant varier de 10⁻¹ à 10⁻³ environ dans un solvant acceptable la solution obtenue à l'étape ii), puis dynamiser par succussion ladite solution diluée, et
iv) répéter l'étape iii) jusqu'à obtention d'une solution diluée à au moins 10⁻⁸ environ et dynamisée.

Par l'expression "de type homéopathique" utilisée selon l'invention, on désigne une solution fortement diluée obtenue par la mise en oeuvre de manipulations classiques en homéopathie, ces techniques étant notamment décrites dans la pharmacopée française.

Ainsi, les techniques pour réaliser les dilutions nécessaires à l'obtention de la solution selon l'invention correspondent aux techniques usuelles de fabrication de préparations homéopathiques. Les dilutions effectuées peuvent être notamment de type hahnémanien, le flacon étant changé à chaque étape de dilution ou de type korsakovien, le même flacon étant utilisé dans toutes les étapes de dilution du procédé de préparation.

De même, la succussion est l'agitation appliquée lors des dilutions afin d'obtenir non seulement des solutions homogènes mais surtout de mettre en contact le solvant avec le principe actif de départ et, ainsi, obtenir une solution dynamisée.

Par l'expression "individu d'une espèce donnée", on entend un individu appartenant au règne végétal ou animal et en particulier un être humain.

Le terme "ADN" désigne bien entendu de l'acide désoxyribonucléique et le terme "ARN" de l'acide ribonucléique.

Selon un mode de réalisation préféré du procédé selon l'invention, l'acide nucléique mis en solution dans l'étape (i) a une longueur au moins égale à 15 nucléotides.

Le produit mis en solution dans le procédé selon l'invention est un acide nucléique spécifique, en particulier un acide nucléique simple brin. Cet acide nucléique simple brin est dit spécifique dans la mesure où sa séquence est sensiblement analogue à celle d'un polynucléotide choisi. Par le terme "analogue", on entend la même succession de bases puriques et pyrimidiques que dans le polynucléotide choisi sous réserve, bien entendu, de la transposition des désoxyribonucléosides en ribonucléosides correspondants et de la thymidine en uracile lorsque le polynucléotide est de l'ADN et vice-versa lorsque le polynucléotide est de l'ARN.

Outre la succession de bases proprement dites, les acides nucléiques présentent à l'une de leurs extrémités une fonction hydroxy en position 3' du dernier nucléotide présent à cette extrémité et une fonction phosphate en position 5' du dernier nucléotide de l'autre extrémité. Ces extrémités sont ainsi classiquement désignées sous les termes "extrémité 3'" et "extrémité 5"'. Ceci confère donc une orientation à la molécule d'acide nucléique. Par le terme "analogue", on entend également la conservation de cette orientation.

L'acide nucléique mis en solution lors de l'étape i) du procédé selon l'invention peut être un acide nucléique naturel ou de synthèse.

Néanmoins, on utilise de préférence dans le procédé selon l'invention un acide nucléique de synthèse, celui-ci étant ainsi sensiblement exempt d'impuretés plus ou moins indésirables.

Dans l'étape iv) du procédé selon l'invention, on peut répéter l'étape iii) autant de fois que désiré pour obtenir des solutions dynamisées dites de type homéopathique, et en particulier de telle sorte que la dilution de la solution soit au moins de 10⁻⁸.

On répète toutefois de préférence l'étape iii) dans l'étape iv) de sorte que la solution soit diluée à 10⁻¹⁶ environ et dynamisée.

Selon un mode de réalisation particulier du procédé selon l'invention, dans l'étape iv), l'étape iii) est répétée jusqu'à obtention d'une solution diluée à 10⁻³⁶ environ et dynamisée.

Le solvant utilisé aux étapes i), iii) et iv) peut être identique ou différent dans les différentes étapes.

Parmi les solvants pharmaceutiquement acceptables utilisables dans le procédé selon l'invention, on peut citer notamment l'eau, l'alcool et les mélanges eau-alcool. Parmi ceux-ci, on utilise de préférence de l'eau purifiée, des mélanges eau purifiée-éthanol comprenant au moins 80 % ou moins de 5 % d'eau en volume. Parmi les eaux purifiées, on peut utiliser en particulier de l'eau distillée et de l'eau désionisée.

La présente invention a en outre pour objet les solutions de type homéopathique obtenues selon le procédé tel que défini précédemment.

La présente invention a également pour objet les médicaments obtenus à partir des solutions de type homéopathique telles que définies précédemment. Ces médicaments peuvent être administrés par différentes voies et notamment par voie orale, y compris sublinguale, par voie parentérale, ainsi que par voie topique.

Selon un mode de réalisation du médicament selon l'invention, celui-ci se présente sous la forme d'une solution constituée essentiellement par la solution de type homéopathique telle que définie précédemment.

Selon un autre mode de réalisation du médicament selon l'invention, celui-ci se présente sous la forme d'une composition obtenue par imprégnation d'un support solide, pharmaceutiquement acceptable, avec une solution de type homéopathique telle que définie précédemment. Comme support pharmaceutiquement acceptable, on peut citer notamment le lactose, le saccharose, leurs mélanges et le kaolin.

Selon un autre mode de réalisation du médicament selon l'invention, celui-ci se présente sous forme d'une composition à application topique obtenue par mélange d'un véhicule pharmaceutiquement acceptable avec une solution de type homéopathique telle que définie précédemment. Parmi ces véhicules, on peut citer notamment l'eau, l'alcool, les huiles, les graisses et leurs mélanges.

Ladite composition peut ainsi se présenter notamment sous forme de lotion, de lait, de crème, de pommade ou de pâte dentifrice.

La posologie selon laquelle le médicament est administré dépend bien entendu du type de composition utilisé. Par voie sublinguale, elle est par exemple d'une gélule par jour jusqu'à disparition des symptômes, ladite gélule contenant 0,4 cm³ d'une poudre obtenue à partir d' 1 ml de solution de type homéopathique telle que définie précédemment et de 100 g de lactose, et devant être ouverte dans la bouche.

La posologie peut en outre varier au cours du traitement comme par exemple dans le cas de traitement d'un cancer, où l'on distingue une phase dite "d'attaque", durant généralement plus de 8 mois et en particulier de 8 à 12 mois, au cours de laquelle la posologie est de l'ordre d'une gélule par jour ou tous les deux jours, d'une phase dite "d'entretien", durant environ 5 ans, au cours de laquelle la posologie est de l'ordre d'une gélule tous les cinq jours.

La présente invention a également pour objet l'utilisation d'une solution diluée telle qu'obtenue selon l'un quelconque des modes de réalisation du procédé de préparation décrit précédemment dans la préparation de compositions de type homéopathique destinées à traiter ou à prévenir une maladie infectieuse ou impliquant le dysfonctionnement d'un gène chez un individu d'une espèce donnée.

Par maladie infectieuse, on entend toute maladie véhiculée par un agent infectieux quelconque, saprophyte ou pathogène, de type eucaryote, comme notamment un champignon ou un parasite, ou procaryote, comme notamment des bactéries ou des virus.

Comme mentionné précédemment, on utilise, dans la préparation d'une composition de type homéopathique destinée à traiter une maladie infectieuse, une solution diluée telle qu'obtenue par le procédé décrit ci-dessus dans lequel ledit acide nucléique a une séquence sensiblement analogue à une partie du matériel génomique de l'agent infectieux ou à son produit de transcription inverse.

La partie du matériel génomique de l'agent infectieux ou son produit de transcription inverse est choisie de préférence parmi celles intervenant dans la capacité de l'agent infectieux à infecter son hôte, celles intervenant dans la croissance de l'agent infectieux ou bien encore dans sa multiplication.

Selon le type d'agent infectieux, le matériel génomique peut être sous forme d'ADN double brin, par exemple lorsque l'agent infectieux est une bactérie ou un virus de classe I, ou sous forme d'ADN simple brin par exemple lorsque l'agent infectieux est un virus de classe II, ou sous forme d'ARN double brin par exemple lorsque l'agent infectieux est un virus de classe III, ou encore sous forme d'ARN simple brin lorsque l'agent infectieux est un virus de classe IV, V ou VI (selon la classification de Baltimore). Une attention toute particulière doit être portée aux virus de classe VI, dits rétrovirus, dont le matériel génomique sous forme d'ARN simple brin est de même polarité que l'ARN messager, et dont la multiplication implique une étape de transcription inverse, c'est-à-dire de synthèse d'ADN selon une matrice ARN.

Lorsque le matériel génomique de l'agent infectieux est de l'ADN, l'acide nucléique utilisé est de type ARN et réciproquement.

Lorsque le matériel génomique est bicaténaire, l'acide nucléique est de même orientation que celle du brin matrice de l'information.

On utilise dans la préparation d'une composition de type homéopathique destinée à prévenir une maladie infectieuse, une solution diluée telle qu'obtenue selon le procédé décrit précédemment, dans lequel ledit acide nucléique a de préférence une séquence sensiblement analogue au produit de transcription d'une partie du matériel génomique de l'agent infectieux. L'acide nucléique utilisé est de préférence de type ADN, ceci notamment dans le cas où l'agent infectieux est un eucaryote ou un virus de classe I, II ou VI.

Le terme de maladie impliquant le dysfonctionnement d'un gène désigne bien entendu toute maladie présentant cette caractéristique. Ce dysfonctionnement peut bien entendu être à l'origine de la maladie ou lui être seulement associé. Ceci regroupe notamment des maladies dites de type héréditaire impliquant la présence d'une modification d'une partie du matériel génétique, des maladies de type cancéreux, qu'elles soient dues à la présence d'un oncogène, à l'activation d'un oncogène ou à l'inactivation d'un antioncogène ou bien encore des maladies de type auto-immun.

Dans certains cas, ces dernières peuvent faire suite à une infection et être dès lors considérées à la fois comme des maladies infectieuses et comme des maladies impliquant le dysfonctionnement d'un gène.

On utilise dans la préparation d'une composition de type homéopathique destinée à traiter des maladies impliquant le dysfonctionnement d'un gène, une solution diluée obtenue selon le procédé tel que décrit précédemment dans lequel ledit acide nucléique a une séquence sensiblement analogue à une partie de ladite séquence nucléotidique qui est liée au dysfonctionnement et qui est une séquence présente dans ledit gène ou dans une région de régulation dudit gène.

En effet, le dysfonctionnement dudit gène peut être dû à une modification du gène proprement dit ou à une modification d'une région de régulation dudit gène ayant pour effet d'activer de façon anormale ledit gène ou bien de l'inhiber.

Dans ce cas là, ledit acide nucléique utilisé est bien entendu de l'ARN.

On utilise dans la préparation d'une composition de type homéopathique destinée à prévenir une maladie impliquant le dysfonctionnement d'un gène, une solution diluée obtenue selon le procédé tel que décrit précédemment dans lequel ledit acide nucléique a une séquence sensiblement analogue à une partie du produit de transcription de ladite séquence qui est liée au dysfonctionnement et qui est une séquence présente dans ledit gène ou dans une région de régulation dudit gène.

L'acide nucléique utilisé dans ce cas est bien entendu de l'ADN.

L'utilisation de ces compositions dans un traitement préventif est tout particulièrement préconisée dans le cas d'individus ayant un comportement dit à risques, c'est-à-dire par exemple évoluant dans un environnement mutagène tel qu'en présence de rayons X ou de composés chimiques mutagènes, ou ayant une prédisposition à développer ce type de maladies.

Les exemples suivants illustrent l'invention.

### EXEMPLE 1 : Procédé de préparation d'une solution de type homéopathique

On introduit dans un tube 10 ml d'eau distillée puis 7 µg de l'acide nucléique de séquence suivante : celle-ci étant sensiblement analogue à la séquence d'une partie du gène du virus herpès simplex de type I.

On dynamise alors la solution obtenue précédemment par succussion. On rappelle que la dynamisation par succussion consiste principalement à agiter la solution, ceci pouvant être réalisé notamment par une centaine de secousses.

Puis, on prélève 0,1 ml de la solution obtenue précédemment, que l'on introduit ensuite dans 9,9 ml d'eau distillée et répète ces opérations de dilution et de dynamisation jusqu'à obtention d'une solution diluée à 10⁻³⁶ et dynamisée.

Cette solution est destinée à traiter l'herpès de type I.

On prépare, de la même façon, une solution contenant une séquence analogue, mais constituée d'ADN (remplacement de U par T). On obtient une solution qui est utilisable dans la prévention de l'herpès de type I.

### EXEMPLE 2 : Solution de type homéopathique destinée à traiter l'hépatite de type B.

Selon le même mode de préparation que décrit précédemment, on prépare une solution à partir de 7,35 µg de l'acide nucléique de séquence suivante : celle-ci étant sensiblement analogue à la séquence d'une partie de gène du virus de l'hépatite de type B.

### EXEMPLE 3 : Solution de type homéopathique destinée à traiter l'hépatite de type A.

Selon le même procédé que celui décrit à l'exemple 1, on prépare une solution avec 7 µg de l'acide nucléique de séquence suivante : celle-ci étant sensiblement analogue à la séquence d'une partie de gène du virus de l'hépatite de type A.

### EXEMPLE 4 : Solution de type homéopathique destinée à traiter l'hépatite de type C.

Selon le même procédé que celui décrit à l'exemple 1, on a préparé une solution de type homéopathique avec 5,6 µg de l'acide nucléique de séquence suivante : celle-ci étant sensiblement analogue à la séquence d'une partie de gène du virus de l'hépatite de type C.

### EXEMPLE 5 : Solution de type homéopathique destinée à traiter un cancer impliquant le dysfonctionnement d'un oncogène

Selon le même procédé que celui décrit à l'exemple 1, on a préparé une solution de type homéopathique destinée à traiter un cancer impliquant le dysfonctionnement d'un oncogène à l'aide de 7 µg de l'acide nucléique de séquence suivante : celle-ci étant sensiblement analogue à une partie de la séquence d'un oncogène.

### EXEMPLE 6 : Solution de type homéopathique destinée à traiter un cancer impliquant le dysfonctionnement d'un oncogène

Selon le même procédé que celui décrit à l'exemple 1, on a préparé une solution de type homéopathique à l'aide de 7 µg de l'acide nucléique de séquence suivante : celle-ci étant sensiblement analogue à une partie de la séquence d'un oncogène.

### EXEMPLE 7 : Solution de type homéopathique destinée à traiter un cancer impliquant le dysfonctionnement d'un oncogène

Selon le même procédé que celui décrit à l'exemple 1, on a préparé une solution de type homéopathique à l'aide de 7 µg de l'acide nucléique de séquence suivante : celle-ci étant sensiblement analogue à une partie de la séquence d'un oncogène.

### EXEMPLE 8 : Solution de type homéopathique destinée à traiter un cancer impliquant le dysfonctionnement d'un oncogène

Selon le même procédé que celui décrit à l'exemple 1, on a préparé une solution de type homéopathique à l'aide de 7 µg de l'acide nucléique de séquence suivante : celle-ci étant sensiblement analogue à une partie de la séquence d'un oncogène.

### EXEMPLE 9 : Préparation d'une poudre destinée à une formulation en gélules pour administration sublinguale.

On répartit 100 g de lactose, puis ajoute 1 ml de la solution obtenue à l'exemple 1. On agite le mélange pendant 5 min. jusqu'à obtention d'une imprégnation homogène, celle-ci pouvant être en particulier estimée par suivi de la répartition de bleu de méthylène. On sèche alors le mélange à une température inférieure à 40°C, puis répartit la poudre obtenue en gélules à ouvrir sous la langue, utilisables dans le traitement de l'herpès de type I.

De façon analogue à celle décrite précédemment, on a préparé des gélules à l'aide des solutions obtenues aux exemples 2 à 8.

On donne ci-après un exemple d'administration.

En administrant à un patient atteint d'hépatite virale de type B, une fois par jour pendant 6 mois, une gélule contenant 0,4 cm³ de poudre obtenue selon le même procédé que décrit à l'exemple 9 mais à partir de la solution de type homéopathique obtenue à l'exemple 2, on observe une disparition des symptômes présentés initialement par ledit patient.

### ETUDE CLINIQUE

### Traitement de malades atteints du Syndrome d'ImmunoDéficience Acquise (SIDA)

Selon le même procédé que celui décrit à l'exemple 1, on a préparé une solution de type homéopathique à l'aide de 7 µg de l'acide nucléique de séquence suivante : celle-ci étant sensiblement analogue à une partie de la séquence du génome du virus HIV1, tel que décrit par Oucy et al., Sciences, 1988, 15 Janvier, 239, 295-297.

Cette solution a été utilisée pour traiter 9 individus infectés par le virus d'immunodéficience humaine (VIH) au stade IV de la maladie selon la classification du CDC d'Atlanta.

Tous les individus présentaient avant le début du traitement et depuis au moins 6 mois soit une baisse soit une stabilité (dans ce cas une variation maximale de 10 % a été admise) de leur nombre absolu de lymphocytes T4.

Après traitement de ces individus pendant 7 mois , à raison d'une prise quotidienne de 5 gouttes de la solution précitée 15 minutes avant un repas, on a obtenu les résultats suivants :
a) Evolution de l'état général des individus appréciée par l'observation i) de la diminution puis de la disparition de la fièvre considérée initialement comme persistante, ii) de la diminution notable de la fréquence des maladies associées et iii) de la diminution notable du nombre ou de la taille des ganglions :
   . Amélioration notable : 7/9
   . Maintien de l'état général de départ : 1/9
   . Baisse de l'état général : 1/9
b) Evolution du poids :
   . Augmentation : 7/9
   . Maintien du poids de départ : 1/9
   . Baisse : 1/9
c) Evolution du taux de lymphocytes T4 circulants :
   Par convention, le nombre absolu de lymphocytes T4 déterminé juste avant le début de la prise a été fixé à une valeur de 100 % chez chaque patient.
   L'évolution a été mesurée en pourcentage d'augmentation ou de baisse par rapport à ces 100 % de départ.
   . Augmentation du taux : 7/9
   . Maintien du taux de départ : 2/9
   . Baisse du taux : 0/9
   Conclusion : Quelque soit le paramètre étudié (évolution de l'état général, évolution du poids ou évolution du nombre de lymphocytes T4 circulants), on observe que plus de la moitié des patients traités présente une amélioration.

On constate donc que l'administration d'une solution de type homéopathique selon l'invention à des individus infectés par le VIH, se traduit par un effet bénéfique pour ceux-ci.

### LISTE DES SEQUENCES

(1) INFORMATIONS GENERALES
   (i) DEPOSANT:
      (A) NOM : Société Anonyme dite : LABO'LIFE
      (B) RUE : La Rambourgère Sainte Marie
      (C) VILLE : La,Chapelle Thireuil
      (E) PAYS : FRANCE
      (F) CODE POSTAL : 79160
      (G) TELEPHONE : (33) 49 04 22 12
      (H) TELECOPIE : (33) 49 04 22 13
   (ii) TITRE DE L'INVENTION : Solutions de type homéopathique contenant un acide nucléique utilisables notamment dans la prévention ou le traitement de maladies infectieuses ou de maladies impliquant le dysfonctionnement d'un gène.
   (iii) NOMBRE DE SEQUENCES : 9
   (iv) FORME LISIBLE PAR ORDINATEUR :
      (A) TYPE DE SUPPORT : Disquette 3,50 pouces, 1,4 Mo de mémoire
      (B) ORDINATEUR : PC compatible
      (C) SYSTEME D'EXPLOITATION : WINDOWS, Version 3.10
      (D) LOGICIEL : WORD, Version 6.0
(2) INFORMATION POUR LA SEQ ID NO : 1 :
   (i) CARACTERISTIQUE DE LA SEQUENCE :
      (A) LONGUEUR : 20
      (B) TYPE : acide nucléique
      (C) NOMBRE DE BRIN : simple
      (D) CONFIGURATION : linéaire
   (ii) TYPE DE MOLECULE : ARN (synthèse chimique)
   (xi) DESCRIPTION DE LA SEQUENCE : SEQ ID NO : 1 :
   AGCAUACGCC GACCUCCCAG 20
(2) INFORMATION POUR LA SEQ ID NO : 2 :
   (i) CARACTERISTIQUE DE LA SEQUENCE :
      (A) LONGUEUR : 21
      (B) TYPE : acide nucléique
      (C) NOMBRE DE BRIN : simple
      (D) CONFIGURATION : linéaire
   (ii) TYPE DE MOLECULE : ARN (synthèse chimique)
   (xi) DESCRIPTION DE LA SEQUENCE : SEQ ID NO : 2 :
   CUAUCCCCGU AAACCACCAG A 21
(2) INFORMATION POUR LA SEQ ID NO : 3 :
   (i) CARACTERISTIQUE DE LA SEQUENCE :
      (A) LONGUEUR : 20
      (B) TYPE : acide nucléique
      (C) NOMBRE DE BRIN : simple
      (D) CONFIGURATION : linéaire
   (ii) TYPE DE MOLECULE : ADN (synthèse chimique)
   (xi) DESCRIPTION DE LA SEQUENCE : SEQ ID NO : 3 :
   AGACATCTTT GACAACAACT 20
(2) INFORMATION POUR LA SEQ ID NO : 4 :
   (i) CARACTERISTIQUE DE LA SEQUENCE :
      (A) LONGUEUR : 16
      (B) TYPE : acide nucléique
      (C) NOMBRE DE BRIN : simple
      (D) CONFIGURATION : linéaire
   (ii) TYPE DE MOLECULE : ADN (synthèse chimique)
   (xi) DESCRIPTION DE LA SEQUENCE : SEQ ID NO : 4 :
   TATGTAGTAC TGTACG 16
(2) INFORMATION POUR LA SEQ ID NO : 5 :
   (i) CARACTERISTIQUE DE LA SEQUENCE :
      (A) LONGUEUR: 20
      (B) TYPE : acide nucléique
      (C) NOMBRE DE BRIN : simple
      (D) CONFIGURATION : linéaire
   (ii) TYPE DE MOLECULE: ARN (synthèse chimique)
   (xi) DESCRIPTION DE LA SEQUENCE: SEQ ID NO : 5 :
   AAGUCGUCCA ACCGUUAGAG 20
(2) INFORMATION POUR LA SEQ ID NO : 6 :
   (i) CARACTERISTIQUE DE LA SEQUENCE:
      (A) LONGUEUR : 20
      (B) TYPE : acide nucléique
      (C) NOMBRE DE BRIN : simple
      (D) CONFIGURATION : linéaire
   (ii) TYPE DE MOLECULE : ARN (synthèse chimique)
   (xi) DESCRIPTION DE LA SEQUENCE : SEQ ID NO : 6 :
   CCACAACAUU CAAGGUCACG 20
(2) INFORMATION POUR LA SEQ ID NO : 7 :
   (i) CARACTERISTIQUE DE LA SEQUENCE:
      (A) LONGUEUR : 20
      (B) TYPE : acide nucléique
      (C) NOMBRE DE BRIN : simple
      (D) CONFIGURATION : linéaire
   (ii) TYPE DE MOLECULE: ARN (synthèse chimique)
   (xi) DESCRIPTION DE LA SEQUENCE : SEQ ID NO : 7 :
   UUGCAUCCUC CCGCUCGUCU 20
(2) INFORMATION POUR LA SEQ ID NO : 8 :
   (i) CARACTERISTIQUE DE LA SEQUENCE :
      (A) LONGUEUR : 23
      (B) TYPE : acide nucléique
      (C) NOMBRE DE BRIN : simple
      (D) CONFIGURATION : linéaire
   (ii) TYPE DE MOLECULE : ARN (synthèse chimique)
   (xi) DESCRIPTION DE LA SEQUENCE : SEQ ID NO : 8 :
   CCUCGGACGG AGAAAAGGUG UCU 23
(2) INFORMATION POUR LA SEQ ID NO : 9 :
   (i) CARACTERISTIQUE DE LA SEQUENCE :
      (A) LONGUEUR: 21
      (B) TYPE : acide nucléique
      (C) NOMBRE DE BRIN : simple
      (D) CONFIGURATION : linéaire
   (ii) TYPE DE MOLECULE : ADN (synthèse chimique)
   (xi) DESCRIPTION DE LA SEQUENCE : SEQ ID NO : 9 :
   CCACAGTGTG AGTTGCAACA G 21

## Revendications

1. Procédé de préparation d'une solution de type homéopathique destinée à traiter ou à prévenir une maladie infectieuse ou impliquant le dysfonctionnement d'un gène, chez un individu d'une espèce donnée, **caractérisé par le fait que** ledit procédé comprend les étapes consistant à :
i) mettre en solution dans un solvant pharmaceutiquement acceptable, dans une proportion correspondant à environ une nanomole pour 10 ml de solvant, un acide nucléique simple brin de longueur au moins égale à 10 nucléotides, de séquence sensiblement analogue à celle d'un polynucléotide choisi soit parmi une partie du matériel génomique de l'agent infectieux, le produit de transcription de ladite partie ou le cas échéant le produit de transcription inverse de ladite partie, lorsque ladite maladie à traiter ou à prévenir est du type infectieux, soit parmi une partie du matériel génétique de ladite espèce, comprenant une partie de la séquence nucléotidique liée audit dysfonctionnement ou une partie du produit de transcription de ladite séquence liée au dysfonctionnement, lorsque ladite maladie à traiter ou à prévenir implique le dysfonctionnement d'un gène, ledit acide nucléique étant de l'ADN lorsque ledit polynucléotide est de l'ARN ou étant de l'ARN lorsque ledit polynucléotide est de l'ADN, à condition que lorsque l'agent infectieux est un virus à ARN, ledit acide nucléique ne soit pas l'ARN viral ou l'un de ses fragments et que lorsque l'agent infectieux est un virus à ADN, ledit acide nucléique ne soit pas l'ADN viral ou l'un de ses fragments,
ii) dynamiser ladite solution par succussion,
iii) diluer d'un facteur de 10⁻¹ à 10⁻³ environ dans un solvant acceptable la solution obtenue à l'étape ii), puis dynamiser par succussion ladite solution diluée, et
iv) répéter l'étape iii) jusqu'à obtention d'une solution diluée à au moins 10⁻⁸ environ et dynamisée.

2. Procédé selon la revendication 1, **caractérisé par le fait que** ledit acide nucléique a une longueur au moins égale à 15 nucléotides.

3. Procédé selon l'une quelconque des revendications précédentes, **caractérisé par le fait que** ledit acide nucléique est un acide nucléique de synthèse.

4. Procédé selon l'une quelconque des revendications précédentes, **caractérisé par le fait que** dans l'étape iv), l'étape iii) est répétée jusqu'à l'obtention d'une solution diluée à environ 10⁻¹⁶ et dynamisée.

5. Procédé selon la revendication 4, **caractérisé par le fait que** dans l'étape iv), l'étape iii) est répétée jusqu'à obtention d'une solution diluée à environ 10⁻³⁶ et dynamisée.

6. Procédé selon l'une quelconque des revendications précédentes, **caractérisé par le fait que** ledit solvant est choisi parmi l'eau, l'alcool et les mélanges eau-alcool.

7. Solution de type homéopatique susceptible d'être obtenue par le procédé selon l'une quelconque des revendications précédentes.

8. Médicament **caractérisé par le fait qu'**il se présente sous la forme d'une solution constituée essentiellement par la solution de type homéopathique telle que définie dans la revendication 7.

9. Médicament **caractérisé par le fait qu'**il se présente sous la forme d'une composition obtenue par imprégnation d'un support solide pharmaceutiquement acceptable avec une solution de type homéopathique telle que définie dans la revendication 7.

10. Médicament selon la revendication 9, **caractérisé par le fait que** ledit support solide pharmaceutiquement acceptable est choisi parmi le lactose, le saccharose, leurs mélanges et le kaolin.

11. Médicament **caractérisé par le fait qu'**il se présente sous forme d'une composition à application topique obtenue par mélange d'un véhicule pharmaceutiquement acceptable avec une solution de type homéopathique telle que définie dans la revendication 7.

12. Médicament selon la revendication 11, **caractérisé par le fait que** ledit véhicule pharmaceutiquement acceptable est choisi parmi l'eau, l'alcool, les huiles, les graisses et leurs mélanges.

13. Utilisation de la solution diluée telle que définie dans la revendication 7 dans la préparation d'une composition de type homéopathique destinée à traiter une maladie infectieuse, **caractérisée par le fait que** ledit acide nucléique a une séquence sensiblement analogue à une partie du matériel génomique de l'agent infectieux ou à son produit de transcription inverse, ladite partie du matériel génomique ou son produit de transcription inverse étant choisi parmi les parties du matériel génomique ou leurs produits de transcription inverse intervenant dans la capacité dudit agent infectieux à infecter son hôte, lesdites parties ou leurs produits de transcription inverse intervenant dans la croissance dudit agent infectieux et lesdites parties ou leurs produits de transcription inverse intervenant dans la multiplication dudit agent infectieux.

14. Utilisation de la solution diluée telle que definie dans la revendication 7 dans la préparation d'une composition de type homéopathique destinée à prévenir une maladie infectieuse, **caractérisée par le fait que** ledit acide nucléique a une séquence sensiblement analogue au produit de transcription d'une partie du matériel génomique de l'agent infectieux.

15. Utilisation de la solution diluée telle que définie dans la revendication 7 dans la préparation d'une composition de type homéopathique destinée à traiter une maladie impliquant le dysfonctionnement d'un gène, **caractérisée par le fait que** ledit acide nucléique a une séquence sensiblement analogue à une partie de ladite séquence nucléotidique liée au dysfonctionnement qui est une séquence présente dans ledit gène ou dans une région de régulation dudit gène.

16. Utilisation de la solution diluée telle que définie dans la revendication 7 dans la préparation d'une composition de type homéopathique destinée à prévenir une maladie impliquant le dysfonctionnement d'un gène, **caractérisée par le fait que** ledit acide nucléique a une séquence sensiblement analogue à une partie du produit de transcription de ladite séquence nucléotidique liée au dysfonctionnement qui est une séquence présente dans ledit gène ou dans une région de régulation dudit gène.

## Patentansprüche

1. Verfahren zur Herstellung einer homöopathischen Lösung, bestimmt zur Behandlung oder Vorbeugung einer infektiösen Erkrankung oder einer Erkrankung, die die Dysfunktion eines Gens voraussetzt, bei einem Individuum einer bestimmten Spezies, **dadurch gekennzeichnet, dass** das Verfahren die Schritte umfasst, bestehend aus:
i) In-Lösung-bringen in einem annehmbaren pharmazeutischen Lösungsmittel in einer Menge, entsprechend etwa einem Nanomol auf 10 ml Lösungsmittel, einer einsträngigen Nukleinsäure einer Länge von mindestens 10 Nukleotiden mit einer Sequenz merklich analog derjenigen eines Polynukleotids, ausgewählt entweder unter einem Teil des genomischen Materials des infektiösen Mittels, dem Transkriptionsprodukt dieses Teils oder gegebenenfalls dem inversen Transkriptionsprodukt dieses Teils, wenn die zu behandelnde oder vorzubeugende Erkrankung vom infektiösen Typ ist, oder unter einem Teil des genetischen Materials dieser Spezies, enthaltend einen Teil der Nukleotidsequenz, die mit der Dysfunktion verbunden ist, oder einem Teil des Transkriptionsprodukts dieser mit der Dysfunktion verbundenen Sequenz, wenn die zu behandelnde oder vorzubeugende Erkrankung die Dysfunktion eines Gens voraussetzt, wobei die Nukleinsäure eine DNS ist, wenn das Polynukleotid eine RNS ist, oder eine RNS ist, wenn das Polynukleotid eine DNS ist, unter der Bedingung, dass dann, wenn das infektiöse Mittel ein RNS-Virus ist, es sich bei der Nukleinsäure nicht um die virale RNS oder eines ihrer Fragmente handelt, und dann, wenn das infektiöse Mittel ein DNS-Virus ist, es sich bei der Nukleinsäure nicht um eine virale DNS oder eines ihrer Fragmente handelt,
ii) Dynamisieren der Lösung durch Schütteln,
iii) Verdünnen der in Schritt (ii) erhaltenen Lösung um einen Faktor von etwa 10⁻¹ bis 10⁻³, anschließendes Dynamisieren der verdünnten Lösung durch Schütteln und
iv) Wiederholen von Schritt iii) bis zum Erhalt einer verdünnten Lösung von mindestens etwa 10⁻⁸ und Dynamisieren.

2. Verfahrung gemäß Anspruch 1, **dadurch gekennzeichnet, dass** die Nukleinsäure eine Länge von mindestens gleich 15 Nukleotiden aufweist.

3. Verfahren gemäß einem der vorstehenden Ansprüche, **dadurch gekennzeichnet dass** die Nukleinsäure eine synthetische Nukleinsäure ist.

4. Verfahren gemäß einem der vorstehenden Ansprüche, **dadurch gekennzeichnet, dass** in Schritt iv) Schritt iii) bis zum Erhalt einer auf etwa 10⁻¹⁶ verdünnten und dynamisierten Lösung wiederholt wird.

5. Verfahren gemäß Anspruch 4, **dadurch gekennzeichnet, dass** in Schritt iv) Schritt iii) bis zum Erhalt einer auf etwa 10⁻³⁶ verdünnten und dynamisierten Lösung wiederholt wird.

6. Verfahren gemäß einer der vorstehenden Ansprüche, **dadurch gekennzeichnet, dass** das Lösungsmittel ausgewählt ist unter Wasser, Alkohol und Wasser/Alkohol-Mischungen.

7. Lösung vom homöopathischen Typ, die über ein Verfahren gemäß einem der vorstehenden Ansprüche erhalten werden kann.

8. Medikament, **dadurch gekennzeichnet, dass** es in Form einer Lösung vorliegt, bestehend im Wesentlichen aus der Lösung vom homöopathischen Typ, wie sie im Anspruch 7 definiert ist.

9. Medikament, **dadurch gekennzeichnet, dass** es in Form einer Zubereitung vorliegt, erhalten durch Imprägnieren eines festen, pharmazeutisch annehmbaren Trägers mit einer homöopathischen Lösung, wie sie im Anspruch 7 definiert ist.

10. Medikament gemäß Anspruch 9, **dadurch gekennzeichnet, dass** der feste, pharmazeutisch annehmbare Träger ausgewählt ist unter Lactose, Saccharose, deren Mischungen und Kaolin.

11. Medikament, **dadurch gekennzeichnet, dass** es in Form einer Zubereitung für die topische Anwendung vorliegt, erhalten durch Mischen eines pharmazeutisch annehmbaren Trägers mit einer homöopathischen Lösung, wie sie in Anspruch 7 definiert ist.

12. Medikament gemäß Anspruch 11, **dadurch gekennzeichnet, dass** der pharmazeutisch annehmbare Träger ausgewählt ist unter Wasser, Alkohol, den Ölen, den Fetten und deren Mischungen.

13. Verwendung der verdünnten Lösung, wie sie in Anspruch 7 definiert ist, bei der Herstellung einer homöopathischen Zubereitung, bestimmt zur Behandlung einer infektiösen Erkrankung, **dadurch gekennzeichnet, dass** die Nukleinsäure eine Sequenz aufweist, merklich analog einem Teil des genomischen Materials des infektiösen Mittels oder deren inversem Transkriptionsprodukt, wobei der Teil des genomischen Materials oder sein inverses Transkriptionprodukt ausgewählt sind unter Teilen des genomischen Materials oder deren inversen Transkriptionsprodukten, die mit der Fähigkeit des infektiösen Mittels seinen Wirt zu infizierern intervenieren, den Teilen oder deren inversen Transkriptionsprodukte, die im Wachstum des infektiösen Mittels intervenieren, und den Teilen oder deren inversen Transkriptionsprodukten, die bei der Multiplikation des infektiösen Mittels intervenieren.

14. Verwendung der verdünnten Lösung, wie sie im Anspruch 7 definiert ist, bei der Herstellung einer homöopathischen Zubereitung, bestimmt zur Vorbeugung einer infektiösen Erkrankung, **dadurch gekennzeichnet, dass** die Nukleinsäure eine dem Transkriptionsprodukt eines Teils des genomischen Materials des infektiösen Mittels merklich homologe Sequenz aufweist.

15. Verwendung der verdünnten Lösung, wie sie im Anspruch 7 definiert ist, bei der Herstellung einer homöopathischen Zubereitung, bestimmt zur Behandlung einer Erkrankung, die die Dysfunktion eines Gens voraussetzt, **dadurch gekennzeichnet, dass** die Nukleinsäure eine Sequenz aufweist, die einem Teil der mit der Dysfunktion verbundenen Nukleotidsequenz merklich analog ist, bei der es sich um eine in dem besagten Gen oder in einem Regulationsbereich des Gens vorhandene Sequenz handelt.

16. Verwendung einer verdünnten Lösung, wie sie in Anspruch 7 definiert ist, bei der Herstellung eine homöopathischen Zubereitung, bestimmt zur Vorbeugung einer Erkrankung, die die Dysfunktion eines Gens voraussetzt, **dadurch gekennzeichnet, dass** die Nukleinsäure eine Sequenz aufweist, die merklich analog ist einem Teil des Transkriptionsprodukts der Nukleotidsequenz, die mit der Dysfunktion verknüpft ist, bei der es sich um eine in dem besagten Gen oder in einem Regulationsbereich des Gens vorhandene Sequenz handelt.

## Claims

1. Method for preparing a homeopathic-type solution intended to treat or prevent an infectious disease or a disease involving the dysfunction of a gene, in an individual of a given species, **characterized in that** said method comprises the steps consisting in:
i) dissolving, in a pharmaceutically acceptable solvent, in a proportion corresponding to approximately one nanomole per 10 ml of solvent, a single-stranded nucleic acid which is at least 10 nucleotides long and which has a sequence substantially analogous to that of a polynucleotide chosen either from a portion of the genomic material of the infectious agent, the product of transcription of said portion or, where appropriate, the product of reverse transcription of said portion, when said disease to be treated or prevented is of the infectious type, or from a portion of the genetic material of said species, comprising a portion of the nucleotide sequence linked to said dysfunction or a portion of the product of transcription of said sequence linked to the dysfunction, when said disease to be treated or prevented involves the dysfunction of a gene, said nucleic acid being DNA when said polynucleotide is RNA or being RNA when said polynucleotide is DNA, on the condition that, when the infectious agent is an RNA virus, said nucleic acid is not the viral RNA or a fragment thereof, and that, when the infectious agent is a DNA virus, said nucleic acid is not the viral DNA or a fragment thereof,
ii) dynamizing said solution by succussion,
iii) diluting the solution obtained in step ii) by a factor of approximately 10⁻¹ to 10⁻³ in an acceptable solvent, then dynamizing said diluted solution by succussion, and
iv) repeating step iii) until a solution which has been diluted to at least approximately 10⁻⁸ and which has been dynamized is obtained.

2. Method according to Claim 1, **characterized in that** said nucleic acid is at least 15 nucleotides long.

3. Method according to any one of the preceding claims, **characterized in that** said nucleic acid is a synthetic nucleic acid.

4. Method according to any one of the preceding claims, **characterized in that**, in step iv), step iii) is repeated until a solution which has been diluted to approximately 10⁻¹⁶ and which has been dynamized is obtained.

5. Method according to Claim 4, **characterized in that**, in step iv), step iii) is repeated until a solution which has been diluted to approximately 10⁻³⁶ and which has been dynamized is obtained.

6. Method according to any one of the preceding claims, **characterized in that** said solvent is chosen from water, alcohol and water/alcohol mixtures.

7. Homeopathic-type solution which can be obtained using the method according to any one of the preceding claims.

8. Medicinal product, **characterized in that** it is in the form of a solution consisting essentially of the homeopathic-type solution as defined in Claim 7.

9. Medicinal product, **characterized in that** it is in the form of a composition obtained by impregnating a pharmaceutically acceptable solid support with a homeopathic-type solution as defined in Claim 7.

10. Medicinal product according to Claim 9, **characterized in that** said pharmaceutically acceptable solid support is chosen from lactose, sucrose, mixtures thereof and kaolin.

11. Medicinal product, **characterized in that** it is in the form of a composition for topical application obtained by mixing a pharmaceutically acceptable vehicle with a homeopathic-type solution as defined in Claim 7.

12. Medicinal product according to Claim 11, **characterized in that** said pharmaceutically acceptable vehicle is chosen from water, alcohol, oils, fats and mixtures thereof.

13. Use of the diluted solution as defined in Claim 7, in the preparation of a homeopathic-type composition intended to treat an infectious disease, **characterized in that** said nucleic acid has a sequence substantially analogous to a portion of the genomic material of the infectious agent, or to the reverse transcription product thereof, said portion of the genomic material, or the reverse transcription product thereof, being chosen from the portions of the genomic material, or the reverse transcription products thereof, which are involved in the capacity of said infectious agent to infect its host, said portions, or the reverse transcription products thereof, being involved in the growth of said infectious agent and said portions, or the reverse transcription products thereof, being involved in the multiplication of said infectious agent.

14. Use of the diluted solution as defined in Claim 7, in the preparation of a homeopathic-type composition intended to prevent an infectious disease, **characterized in that** said nucleic acid has a sequence substantially analogous to the product of transcription of a portion of the genomic material of the infectious agent.

15. Use of the diluted solution as defined in Claim 7, in the preparation of a homeopathic-type composition intended to treat a disease involving the dysfunction of a gene, **characterized in that** said nucleic acid has a sequence substantially analogous to a portion of said nucleotide sequence linked to the dysfunction which is a sequence present in said gene or in a region for regulating said gene.

16. Use of the diluted solution as defined in Claim 7, in the preparation of a homeopathic-type composition intended to prevent a disease involving the dysfunction of a gene, **characterized in that** said nucleic acid has a sequence substantially analogous to a portion of the product of transcription of said nucleotide sequence linked to the dysfunction which is a sequence present in said gene or in a region for regulating said gene.
